# EUROPÄISCHE PATENTANMELDUNG

(11) **EP 4 357 459 A2**
(43) Veröffentlichungstag der Anmeldung: **24.04.2024**
(21) Anmeldenummer: 23201929.9
(22) Anmeldetag: 05.10.2023
(51) Int. Cl.: C12P 5/02

(54) **AUFBEREITUNG EINER FASERHALTIGEN BIOMASSE MIT PANSENSAFT**

(30) Priorität: 21.10.2022 DE 102022127939
(71) Anmelder: Stühmeier, Klaus, 32549 Bad Oeynhausen (DE)
(72) Erfinder: Stühmeier, Klaus, 32549 Bad Oeynhausen (DE)
(74) Vertreter: Ter Meer Steinmeister & Partner

(57) **Zusammenfassung**

Verfahren zum Aufbereiten einer faserhaltigen Biomasse, die einer Biogasanlage (100) für das Erzeugen von Biogas zugeführt werden soll, mit den Schritten: Bereitstellen (S10) einer faserhaltigen Biomasse (1); Sammeln (S12) der faserhaltigen Biomasse (1), wobei ein Beaufschlagen (S14) der faserhaltigen Biomasse (1) mit einem Mittel (14) zum Aufschließen der faserhaltigen Biomasse (1) erfolgt, und wobei die mit dem Mittel (14) zum Aufschließen der faserhaltigen Biomasse beaufschlagte faserhaltige Biomasse (1) zu einer Ansammlung der faserhaltigen Biomasse (1) zusammengeführt wird; Gestatten (S18) eines Einwirkens des Mittels (14) zum Aufschließen der faserhaltigen Biomasse (1) auf die faserhaltige Biomasse (1) während einer Einwirkzeit; Beaufschlagen (S32) der faserhaltigen Biomasse (1) mit Pansensaft (24); und Zuführen (S34) der mit dem Pansensaft (24) beaufschlagten faserhaltigen Biomasse (1) zu der Biogasanlage (100).

## Beschreibung

Die vorliegende Erfindung betrifft ein Verfahren zum Aufbereiten einer faserhaltigen Biomasse, die einer Biogasanlage für das Erzeugen von Biogas zugeführt werden soll.

### Hintergrund

Als "Bio"-Gas wird ein Gasgemisch mit den Hauptkomponenten Methan und Kohlendioxid bezeichnet, das in einem biologischen Prozess entsteht. In einer Biogasanlage wird unter Ausschluss von Sauerstoff aus organischer Masse das Biogas gebildet. Dabei wandeln Bakterien in einem mehrstufigen Prozess beispielsweise Kohlenhydrate, Fette und Proteine in sauerstofffreier Umgebung in Methan und Kohlendioxid um. Ein solcher Prozess ist in der Natur weit verbreitet und findet beispielsweise in Mooren, auf dem Grund von Seen, in Güllegruben sowie im Pansen von Wiederkäuern statt. Beim Umwandeln von organischer Masse zu Biogas in einer Biogasanlage entsteht außerdem Wärme. Das gebildete Gasgemisch besteht überwiegend aus Methan und Kohlendioxid. Daneben können sich im Biogas noch geringe Mengen an Wasserstoff, Schwefelwasserstoff, Ammoniak und anderen Spurengasen befinden. Die Zusammensetzung wird im Wesentlichen von den eingesetzten Substraten, dem Fermentationsverfahren und Einzelheiten der technischen Durchführung beeinflusst.

### Stand der Technik

EP 2740799 A2 beschreibt ein Verfahren zur Verwertung von Biomasse, welche bei der Produktion von Biokraftstoffen aus Algen entsteht. Hierzu wird die Biomasse in einem Reaktor, welcher im Pansen von Wiederkäuern vorkommende Mikroorganismen enthält, zu verwertbaren Produkten verstoffwechselt.

Christoph Griehl, "Stroh im Tank", Junge Wissenschaft 108 // 2016, Seiten 40-51, Verlag: Physikalisch-Technische Bundesanstalt PTB, Braunschweig, abgerufen im Internet unter https://www.junge-wissenschaft.ptb.de/fileadmin/paper/bis_2017/pdf/juwi-108-2016-03.pdf beschreibt, dass Pansenorganismen effektiver als Gülleorganismen cellulosehaltige Substrate wie Stroh zu Biogas abbauen und erwähnt, dass die Cellulosespaltung mit Pansensaft zukünftig auch in pflanzenvergärenden Biogasanlagen Anwendung finden könnte.

### Kurzfassung der Erfindung

Ein Nebenprodukt in der Nahrungs- und Futtermittelproduktion ist Weizenstroh. In Biogasanlagen findet Stroh jedoch kaum Anwendung, da die Vergärung nur unzureichend abläuft. Gerade für landwirtschaftliche Betriebe, in denen viel Stroh anfällt, bietet der Einsatz von Stroh zur Biogaserzeugung eine interessante Alternative. Die Biomasse liegt bereits vor, was sowohl finanzielle Vorteile für die landwirtschaftlichen Betriebe bietet als auch eine größere wirtschaftliche Unabhängigkeit. Eine bessere Nutzung von Stroh ist daher erstrebenswert.

Es ist daher Aufgabe der vorliegenden Erfindung, ein neuartiges Verfahren zu schaffen, welches eine Aufbereitung von Stroh zur Steigerung einer Biogasausbeute einer Biogasanlage ermöglicht.

Diese Aufgabe wird erfindungsgemäß gelöst durch ein Verfahren nach Anspruch 1 zum Aufbereiten einer faserhaltigen Biomasse, die einer Biogasanlage für das Erzeugen von Biogas zugeführt werden soll, wobei das Verfahren die folgenden Schritte umfasst:
Bereitstellen einer faserhaltigen Biomasse;
Sammeln der faserhaltigen Biomasse, wobei ein Beaufschlagen der faserhaltigen Biomasse mit einem Mittel zum Aufschließen der faserhaltigen Biomasse erfolgt, und wobei die mit dem Mittel zum Aufschließen der faserhaltigen Biomasse beaufschlagte faserhaltige Biomasse zu wenigstens einer Ansammlung der faserhaltigen Biomasse zusammengeführt wird;
Gestatten eines Einwirkens des Mittels zum Aufschließen der faserhaltigen Biomasse auf die faserhaltige Biomasse während einer Einwirkzeit;
Beaufschlagen der faserhaltigen Biomasse mit Pansensaft; und
Zuführen der mit dem Pansensaft beaufschlagten faserhaltigen Biomasse zu der Biogasanlage.

Das Sammeln erfolgt vorzugsweise auf einer landwirtschaftlichen Nutzfläche, auf der die faserhaltige Biomasse entstanden ist. Beispielsweise kann das Sammeln mittels einer über die landwirtschaftliche Nutzfläche fahrenden oder gefahrenen Maschine erfolgen, beispielsweise einer Ballenpresse und/oder einem Feldhäcksler. Vorzugsweise erfolgt das Sammeln auf der fahrenden oder gefahrenen Maschine, beispielsweise durch Verpressen der faserhaltigen Biomasse zu Ballen und/oder durch Zusammenführen zu wenigstens einer Ansammlung in einem mitgeführten Behälter und/oder einer mitgeführten Ladefläche. D.h., die bereitgestellte faserhaltige Biomasse wird beispielsweise aufgenommen und mitgeführt. Danach kann beispielsweise ein Absetzen oder Abladen der jeweiligen Ansammlung (z.B. eines Ballens) erfolgen.

Das Sammeln der faserhaltigen Biomasse kann in Ausführungsformen ein Verpressen der faserhaltigen Biomasse zu Ballen umfassen, wobei die mit dem Mittel zum Aufschließen der faserhaltigen Biomasse beaufschlagte faserhaltige Biomasse zu Ballen gepresst wird. In diesem Fall stellen die Ballen die Ansammlungen der faserhaltigen Biomasse dar, und das Pressen zu Ballen stellt das Zusammenführen zu wenigstens einer Ansammlung dar.

Das Sammeln der faserhaltigen Biomasse kann in Ausführungsformen ein Zusammenführen zu wenigstens einer losen Ansammlung der faserhaltigen Biomasse umfassen, beispielsweise zu einer Ansammlung in einem Behälter und/oder auf einer Ladefläche.

Das Sammeln der faserhaltigen Biomasse kann ein Häckseln umfassen, wobei die faserhaltige Biomasse vor und/oder während und/oder nach dem Beaufschlagen mit dem Mittel zum Aufschließen der faserhaltigen Biomasse gehäckselt wird.

Durch das Verfahren wird eine effektive, umweltverträgliche und kostengünstige Aufbereitung einer Biomasse ermöglicht, die sich als Abfallprodukt oder Nebenprodukt in der Nahrungs- und Futtermittelherstellung ergibt, vorzugsweise eine Aufbereitung von Stroh. Indem bereits direkt beim Sammeln der Biomasse ein Beaufschlagen der Biomasse mit einem Mittel zum Aufschließen der faserhaltigen Biomasse erfolgt, ergeben sich verschiedene Vorteile. Zum einen kann die Biomasse effektiv mit dem Mittel zum Aufschließen der faserhaltigen Biomasse aufbereitet werden, da stets nur die Menge an Biomasse, die gerade gesammelt wird, beispielsweise einem Häcksler und/oder einer Ballenpresse zugeführt wird, mit dem Mittel zum Aufschließen der faserhaltigen Biomasse beaufschlagt wird. Durch die gleichmäßige Beaufschlagung der Biomasse kann außerdem die benötigte Menge an dem Mittel zum Aufschließen der faserhaltigen Biomasse reduziert werden. Würde man Stroh beispielsweise stattdessen in einem großen Sammeltank aufbereiten, wäre eine vergleichbar effektive Aufbereitung nicht möglich. Auf Grund der viel größeren Menge an Biomasse müsste für eine vergleichbar gleichmäßige Beaufschlagung der Biomasse die eingesetzte Menge an dem Mittel zum Aufschließen der faserhaltigen Biomasse im Verhältnis zur Biomasse deutlich erhöht werden. Zum anderen ergibt sich ein zeitlicher Vorteil des Verfahrens, da die Biomasse beispielsweise bereits direkt nach dem Bereitstellen auf der landwirtschaftlichen Nutzfläche mit dem Mittel zum Aufschließen der faserhaltigen Biomasse aufbereitet werden kann und nicht erst abtransportiert werden muss. Aber das Mittel zum Aufschließen der faserhaltigen Biomasse kann die Biomasse auch weiterhin aufbereiten, insbesondere in der jeweiligen Ansammlung der faserhaltigen Biomasse, z.B. während einer Lagerung der gepressten Ballen beispielsweise auf der landwirtschaftlichen Nutzfläche oder der gehäckselten Biomasse in einem Transportbehälter, bis es zu einem anschließenden Abtransport der Biomasse kommt, während des Abtransports und/oder während einer möglichen (erneuten) Lagerung der Biomasse nach dem Abtransport. Diese ohnehin anfallenden Schritte können so effektiv als Einwirkzeit des Mittels zum Aufschließen der faserhaltigen Biomasse genutzt werden, bis die faserhaltige Biomasse mit Pansensaft beaufschlagt wird und einer Biogasanlage zur Produktion von Biogas zugeführt wird. Die Menge des verwendeten Mittels zum Aufschließen der faserhaltigen Biomasse und eine Einwirkdauer der Aufbereitung können individuell angepasst werden. Durch die Aufbereitung der faserhaltigen Biomasse durch Pansensaft vor dem Zuführen der faserhaltigen Biomasse zu einer Biogasanlage kann die Effizienz der Biomasse erneut gesteigert werden. Die vorherige Beaufschlagung der faserhaltigen Biomasse mit dem Mittel zum Aufschließen der faserhaltigen Biomasse erleichtert ein Einwirken des Pansensafts. Durch die Beaufschlagung der Biomasse mit Pansensaft vor dem Zuführen zu der Biogasanlage kann außerdem die benötigte Menge an Pansensaft reduziert werden.

Das eigentliche Aufbereiten der bereitgestellten faserhaltigen Biomasse umfasst die Schritte des Sammelns der faserhaltigen Biomasse, wobei das Beaufschlagen der faserhaltigen Biomasse mit dem Mittel zum Aufschließen der faserhaltigen Biomasse erfolgt, und wobei die mit dem Mittel zum Aufschließen der faserhaltigen Biomasse beaufschlagte faserhaltige Biomasse zu einer Ansammlung der faserhaltigen Biomasse zusammengeführt wird. Anschließend erfolgt das Gestatten eines Einwirkens des Mittels zum Aufschließen der faserhaltigen Biomasse auf die faserhaltige Biomasse während einer Einwirkzeit, bevor das Beaufschlagen der faserhaltigen Biomasse mit Pansensaft erfolgt. Das Gestatten des Einwirkens kann ein Ruhenlassen der Ansammlung, z.B. der gepressten Ballen oder der gehäckselten Biomasse, umfassen. Nach dem Beaufschlagen der faserhaltigen Biomasse mit Pansensaft wird die faserhaltige Biomasse der Biogasanlage zugeführt. Die Biogasanlage ist insbesondere eine Biogasanlage zum Erzeugen von Biogas aus der faserhaltigen Biomasse oder zum Erzeugen von Biogas mittels Umwandeln der Biomasse. Die faserhaltige Biomasse wird der Biogasanlage für das Erzeugen von Biogas zugeführt, insbesondere zum Erzeugen von Biogas aus der faserhaltigen Biomasse bzw. unter Umwandlung der faserhaltigen Biomasse.

Als Pansensaft wird eine im Pansen von Wiederkäuern vorkommende flüssige Phase bezeichnet. Mikroorganismen im Pansensaft können als Teil der sogenannten Pansenflora Essigsäurebakterien, Propionsäurebakterien, Buttersäurebakterien und Milchsäurebakterien umfassen, die Kohlenhydrate in Proteine spalten können. Durch das Einwirken der im Pansensaft enthaltenen Pansenflora auf die faserhaltige Biomasse können Bindungen von Strukturkohlenhydraten in der faserhaltigen Biomasse aufgebrochen werden, und die Effektivität der anschließenden Biogaserzeugung kann gesteigert werden. Da Pansensaft als Abfallprodukt in der Schlachtindustrie anfällt, stellt die Verwendung von Pansensaft eine kostengünstige Möglichkeit zur Aufbereitung von faserhaltiger Biomasse dar.

Bevorzugte Ausgestaltungen der Erfindung ergeben sich aus den Unteransprüchen.

Gemäß bevorzugten Ausführungsformen des Verfahrens umfasst das Sammeln der faserhaltigen Biomasse ein Verpressen der faserhaltigen Biomasse zu Ballen, wobei die mit dem Mittel zum Aufschließen der faserhaltigen Biomasse beaufschlagte faserhaltige Biomasse zu Ballen gepresst wird. Dementsprechend wird gemäß einem Aspekt der Erfindung ein Verfahren zur Verfügung gestellt zum Aufbereiten einer faserhaltigen Biomasse, die einer Biogasanlage für das Erzeugen von Biogas zugeführt werden soll, wobei das Verfahren die folgenden Schritte umfasst:
Bereitstellen einer faserhaltigen Biomasse;
Verpressen der faserhaltigen Biomasse zu Ballen, wobei ein Beaufschlagen der faserhaltigen Biomasse mit einem Mittel zum Aufschließen der faserhaltigen Biomasse erfolgt, und wobei die mit dem Mittel zum Aufschließen der faserhaltigen Biomasse beaufschlagte faserhaltige Biomasse zu Ballen gepresst wird;
Gestatten eines Einwirkens des Mittels zum Aufschließen der faserhaltigen Biomasse auf die faserhaltige Biomasse während einer Einwirkzeit;
Beaufschlagen der faserhaltigen Biomasse mit Pansensaft; und
Zuführen der mit dem Pansensaft beaufschlagten faserhaltigen Biomasse zu der Biogasanlage.

Vorzugsweise umfasst das Zuführen ein Zerkleinern der gepressten Ballen, insbesondere ein Zerkleinern der gepressten Ballen an der Biogasanlage, beispielsweise in oder an einer Fördereinrichtung zum Zuführen der faserhaltigen Biomasse zu der Biogasanlage. Vorzugsweise erfolgt das Beaufschlagen der faserhaltige Biomasse mit Pansensaft während des Zerkleinerns der Ballen und/oder nach dem Zerkleinern der Ballen.

Gemäß einer bevorzugten Ausführungsform des Verfahrens erfolgt das Bereitstellen und/oder das Sammeln der faserhaltigen Biomasse auf einer landwirtschaftlichen Nutzfläche, auf der die faserhaltige Biomasse entstanden ist. Dies ermöglicht einen frühzeitigen Beginn des Aufbereitens und daher einen besonders effizienten Ablauf des Aufbereitens.

Vorzugsweise umfasst das Bereitstellen der faserhaltigen Biomasse: Mähen der landwirtschaftlichen Nutzfläche, und/oder Aufnehmen der lose auf der landwirtschaftlichen Nutzfläche liegenden faserhaltigen Biomasse. Beides kann durch entsprechende Maschinen oder Maschinenkomponenten erfolgen, beispielsweise mittels einer über die landwirtschaftliche Nutzfläche fahrenden oder gefahrenen Maschine.

In bevorzugten Ausführungsformen umfasst das Verfahren weiter: Ruhenlassen der wenigstens einen Ansammlung der faserhaltigen Biomasse, z.B. der gehäckselten Biomasse und/oder der gepressten Ballen, um das Einwirken des Mittels zum Aufschließen der faserhaltigen Biomasse auf die faserhaltige Biomasse zu gestatten, bevor der Schritt des Beaufschlagens der faserhaltigen Biomasse mit Pansensaft ausgeführt wird. Das Ruhenlassen kann beispielsweise während der Einwirkzeit erfolgen.

Vorzugsweise umfasst das Verfahren einen Schritt des Transportierens der wenigstens einen Ansammlung der faserhaltigen Biomasse, z.B. der gehäckselten faserhaltigen Biomasse und/oder der gepressten Ballen, zu der Biogasanlage. Das Transportieren kann beispielsweise vor und/oder während und/oder nach der Einwirkzeit oder dem Ruhenlassen ausgeführt werden.

In bevorzugten Ausführungsformen erfolgt das Häckseln der faserhaltigen Biomasse durch einen Feldhäcksler bzw. das Verpressen der faserhaltigen Biomasse zu Ballen durch eine Ballenpresse. Vorzugsweise ist die Ballenpresse eine Rundballenpresse oder eine Quaderballenpresse. Auch andere Ausführungsformen der Ballenpresse sind denkbar.

Vorzugsweise erfolgt das Sammeln mittels einer über die landwirtschaftliche Nutzfläche fahrenden oder gefahrenen Maschine, mit der eine Einrichtung zum Beaufschlagen der faserhaltigen Biomasse mit dem Mittel zum Aufschließen der faserhaltigen Biomasse mitgeführt wird. Beispielsweise kann die Maschine die Einrichtung umfassen. Beispielsweise kann/können der Feldhäcksler und/oder die Ballenpresse eine Einrichtung zum Beaufschlagen der faserhaltigen Biomasse mit dem Mittel zum Aufschließen der faserhaltigen Biomasse umfassen. Es ist auch denkbar, dass die Beaufschlagung der Biomasse durch eine Einrichtung erfolgt, die mit der Maschine, z.B. dem Feldhäcksler und/oder der Ballenpresse, verbunden ist. Die Einrichtung zum Beaufschlagen kann beispielsweise wenigstens eine Düse zum Besprühen, Berieseln und/oder Bespritzen der faserhaltigen Biomasse mit dem Mittel zum Aufschließen der faserhaltigen Biomasse umfassen. Die Einrichtung zum Beaufschlagen erlaubt vorzugsweise eine Dosierung des Mittels zum Aufschließen der faserhaltigen Biomasse. Das Beaufschlagen kann beispielsweise ein Besprühen, Berieseln und/oder Bespritzen der faserhaltigen Biomasse umfassen.

Vorzugsweise erfolgt das Beaufschlagen der faserhaltigen Biomasse mit dem Mittel zum Aufschließen der faserhaltigen Biomasse aus einem an der Maschine, z.B. dem Feldhäcksler und/oder der Ballenpresse, angeordneten und/oder mit der Maschine, z.B. dem Feldhäcksler und/oder der Ballenpresse, mitgeführtem Tank. Beispielsweise kann der Feldhäcksler und/oder die Ballenpresse den Tank umfassen.

Gemäß bevorzugten Ausführungsformen des Verfahrens ist oder umfasst die faserhaltige Biomasse eine Cellulose-haltige Biomasse. Vorzugsweise ist oder umfasst die faserhaltige Biomasse eine Lignocellulose-haltige Biomasse. Als faserhaltige Biomasse kann außer trockenen Halmen von Getreide nach dem Dreschen (kurz als "Stroh" bezeichnet) auch Gras und/oder anderes Erntegut eingesetzt werden, insbesondere nachdem es gemäht und getrocknet wurde, einschließlich Früchten und/oder Strukturbestandteilen der Pflanzen. Besonders bevorzugt ist oder umfasst die faserhaltige Biomasse Stroh.

Gemäß bevorzugten Ausführungsformen des Verfahrens ist das Mittel zum Aufschließen der faserhaltigen Biomasse eine Lauge. Vorzugsweise ist die Lauge eine Natronlauge. Das Mittel zum Aufschließen der faserhaltigen Biomasse kann auch Gülle umfassen.

Gemäß Ausführungsformen des Verfahrens umfasst das Beaufschlagen der faserhaltigen Biomasse und/oder das Zuführen der mit dem Pansensaft beaufschlagten faserhaltigen Biomasse zu der Biogasanlage ein (ggf. zusätzliches) Zerkleinern der faserhaltigen Biomasse. Dies kann das Einwirken des Pansensafts verbessern. Beispielsweise kann beim Zerkleinern der gepressten Ballen die faserhaltige Biomasse (zusätzlich) zerkleinert werden, oder die gehäckselte Biomasse kann zusätzlich zerkleinert werden.

### Kurzbeschreibung der Zeichnungen

Die Erfindung wird im Folgenden beispielhaft anhand der Zeichnung näher erläutert. Es zeigen:
- Fig. 1: eine schematische Darstellung eines Verfahrens zum Aufbereiten von faserhaltiger Biomasse; und
- Fig. 2: eine schematische Darstellung eines Ausführungsbeispiels des Verfahrens.

### Detaillierte Beschreibung der Ausführungsformen

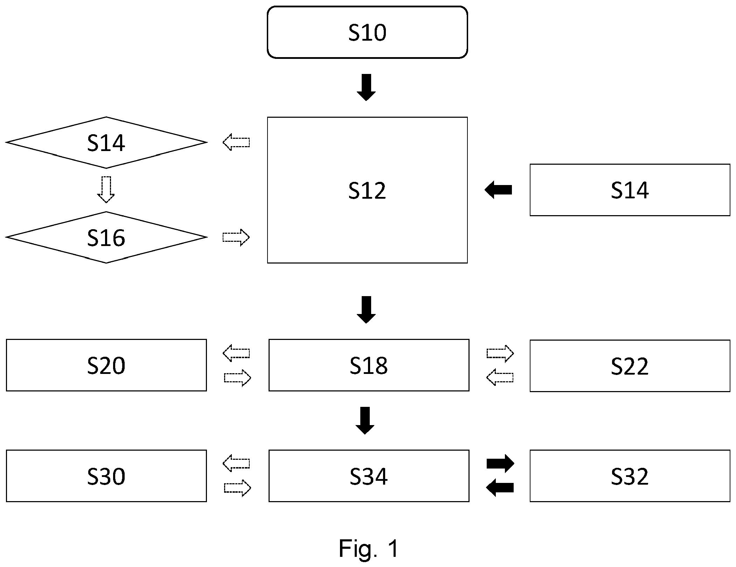

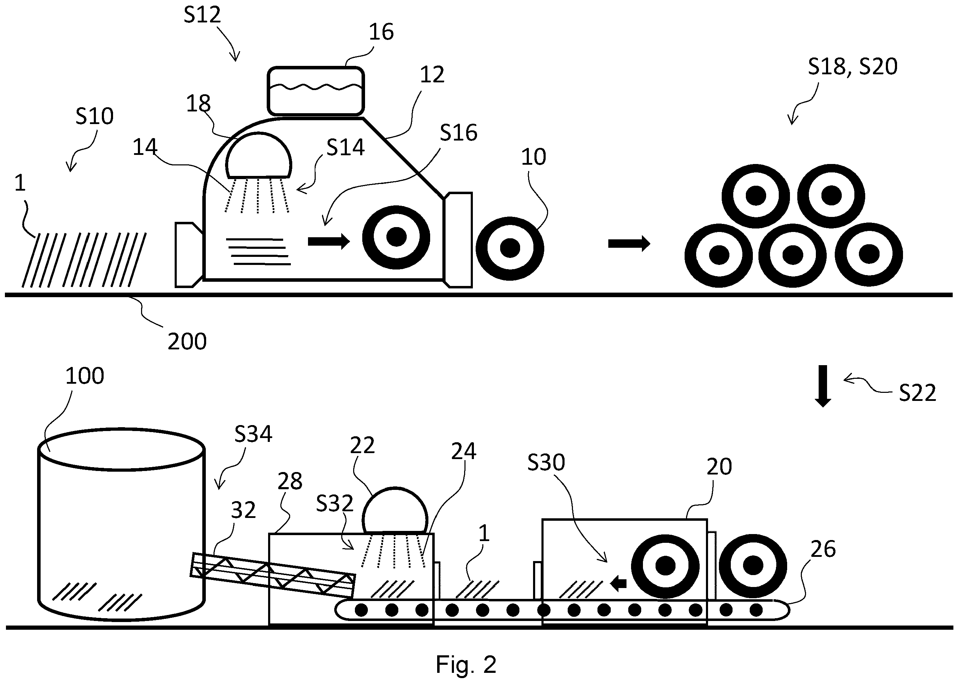

Fig. 1 zeigt eine schematische Darstellung eines Verfahrens zum Aufbereiten von faserhaltiger Biomasse, während Fig. 2 ein schematisch ein Ausführungsbeispiel zeigt. Das Verfahren beginnt mit einem Schritt S10 des Bereitstellens einer faserhaltigen Biomasse 1. In einem nachfolgenden Schritt des Sammelns S12 der faserhaltigen Biomasse 1 erfolgt ein Beaufschlagen S14 der faserhaltigen Biomasse 1 mit einem Mittel 14 zum Aufschließen der faserhaltigen Biomasse 1, wobei die mit dem Mittel 14 zum Aufschließen der faserhaltigen Biomasse beaufschlagte faserhaltige Biomasse 1 zu einer Ansammlung der faserhaltigen Biomasse 1 zusammengeführt wird. Der Schritt S12 des Sammelns kann ein Häckseln S14 der faserhaltigen Biomasse 1 und/oder ein Verpressen S 16 der faserhaltigen Biomasse 1 zu Ballen 10 umfassen.

Anschließend erfolgt ein Schritt S18 des Gestattens eines Einwirkens des Mittels 14 zum Aufschließen der faserhaltigen Biomasse 1 auf die faserhaltige Biomasse 1 während einer Einwirkzeit. Dies kann einen optionalen Schritt S20 des Ruhenlassens der faserhaltige Biomasse 1 zum Gestatten des Einwirkens des Mittels 14 zum Aufschließen der faserhaltigen Biomasse 1 auf die faserhaltige Biomasse 1 umfassen. Das Verfahren umfasst weiter einen Schritt S22 des Transportierens der faserhaltige Biomasse 1 zu einer Biogasanlage 100. Der Schritt S22 des Transportierens kann beispielsweise vor und/oder nach dem Ruhenlassen S 18 der faserhaltige Biomasse 1 ausgeführt werden.

Die gepressten Ballen 10 können anschließend in einem Schritt S30 des Zerkleinerns zerkleinert werden, bevor ein Beaufschlagen S32 der faserhaltige Biomasse 1 mit Pansensaft 24 erfolgt und die mit Pansensaft 24 beaufschlagte faserhaltige Biomasse 1 der Biogasanlage 100 zur Biogasproduktion zugeführt wird (Schritt S34).

Fig. 2 zeigt Ausführungsschritte eines beispielhaften Verfahrens zum Aufbereiten von faserhaltiger Biomasse, wobei beim Bereitstellen S10 eine lose auf einer landwirtschaftlichen Nutzfläche 200 liegende faserhaltige Biomasse 1, die zuvor gemäht wurde, von einer Ballenpresse 12 im Schritt des Sammelns S12 aufgenommen wird. Der Schritt des Sammelns S12 umfasst ein Beaufschlagen S14 der faserhaltigen Biomasse 1 mit einem Mittel 14 zum Aufschließen der faserhaltigen Biomasse 1, und ein Verpressen S16 faserhaltigen Biomasse 1 durch die Ballenpresse 12 in Ballen 10 (im Beispiel Rundballen), wobei während des Verpressens S16 die faserhaltige Biomasse 1 in Ballen 10 gepresst wird und dabei das Beaufschlagen S 14 der Biomasse 1 mit dem Mittel 14 zum Aufschließen der faserhaltigen Biomasse 1, vorzugsweise Natronlauge, erfolgt. Die Biomasse 1 wird hierbei durch in die Ballenpresse 12 integrierte Einrichtung 18 in Form einer oder mehrerer Düsen gleichmäßig mit einer vorgegebenen Menge an dem Mittel 14 zum Aufschließen der faserhaltigen Biomasse 1 beaufschlagt, wobei eine Dosierung des Mittels 14 zum Aufschließen der faserhaltigen Biomasse 1 möglich ist. Der Einrichtung 18 wird das Mittel 14 zum Aufschließen der faserhaltigen Biomasse 1 aus einem an der Ballenpresse 12 angeordneten und/oder mit der Ballenpresse 12 mitgeführtem Tank 16 zur Verfügung gestellt.

Das Mittel 14 zum Aufschließen der faserhaltigen Biomasse 1 bewirkt nach dem Beaufschlagen S14 erste Aktivitäten einer biochemischen Reaktion bereits während und nach dem Verpressen S16 der Biomasse 1 in transportfähige und lagerbare Ballen 10. Dadurch werden langkettige Verbindungen von faserhaltigen Bestandteilen der Biomasse, insbesondere Strukturpolymere, bereits während eines Ruhelassens S 18 der Ballen 10 zum Gestatten S20 eines Einwirkens des Mittels 14 zum Aufschließen der faserhaltigen Biomasse 1 auf die faserhaltige Biomasse 1 während einer Einwirkzeit auf der landwirtschaftlichen Nutzfläche 200 gespalten, sowie während eines Abtransports S22 der Ballen 10 und einer nachfolgenden Lagerung, bevor die Biomasse 1 weiteren Verarbeitungsschritten zugeführt wird. Dadurch kommt es zu einer Verbesserung der Aufbereitung und Erhöhung der Effizienz der Biomasse 1, wobei die Aufbereitung mit dem Mittel 14 zum Aufschließen der faserhaltigen Biomasse 1 keiner zusätzlichen Aufbereitungszeit bedarf, da die ohnehin anfallenden Schritte der Lagerung und des Abtransports der Ballen 10 als Einwirkzeit für das Mittel 14 zum Aufschließen der faserhaltigen Biomasse 1 genutzt werden. Das Aufschließen der Biomasse 1 durch das Mittel 14 zum Aufschließen der faserhaltigen Biomasse 1 wird je nach Konzentration, Menge und Einwirkdauer des Mittels 14 zum Aufschließen der faserhaltigen Biomasse 1 bis zur weiteren Verarbeitung der Ballen 10 bestimmt.

Nach dem Abtransport S22 der Ballen 10 zu einer Biogasanlage 100 werden im Schritt S30 des Zerkleinerns die gepressten Ballen 10 über eine Fördereinrichtung, z.B. ein Förderband 26, in einen Häcksler 20 befördert und im Häcksler 20 zerkleinert. Anschließend wird die Biomasse 1 während eines Aufbereitens in einer Aufbereitungsanlage 28 mit Pansensaft 24 zum Aufbereiten der faserhaltigen Biomasse 1 beaufschlagt (S32), wobei die Aufbereitungsanlage 28 über eine Einrichtung 22 zum Beaufschlagen verfügt. Der Pansensaft 24 bewirkt weitere Abbauaktivitäten einer zweiten chemischen Reaktion zum chemischen Aufschließen der faserhaltigen Biomasse 1, wobei beispielsweise langkettige Verbindungen der Biomasse 1 aufgespalten werden können. Die so aufbereitete faserhaltige Biomasse 1 wird anschließend der Biogasanlage 100 zur Biogasproduktion über eine Fördereinrichtung, beispielsweise über eine Förderschnecke 32, zugeführt (S34), um in an sich bekannten, weiteren Schritten einer Verwertung der faserhaltigen Biomasse 1 Biogas zu erzeugen. Beim Zuführen kann die faserhaltige Biomasse 1 erneut zerkleinert werden, um die Oberfläche der Biomasse 1 zu vergrößern und um die nachfolgenden Abläufe in der Biogasanlage 100 zu optimieren.

## Patentansprüche

1. Verfahren zum Aufbereiten einer faserhaltigen Biomasse, die einer Biogasanlage (100) für das Erzeugen von Biogas zugeführt werden soll, wobei das Verfahren die folgenden Schritte umfasst:
Bereitstellen (S10) einer faserhaltigen Biomasse (1);
Sammeln (S12) der faserhaltigen Biomasse (1), wobei ein Beaufschlagen (S14) der faserhaltigen Biomasse (1) mit einem Mittel (14) zum Aufschließen der faserhaltigen Biomasse (1) erfolgt, und wobei die mit dem Mittel (14) zum Aufschließen der faserhaltigen Biomasse beaufschlagte faserhaltige Biomasse (1) zu wenigstens einer Ansammlung der faserhaltigen Biomasse (1) zusammengeführt wird;
Gestatten (S18) eines Einwirkens des Mittels (14) zum Aufschließen der faserhaltigen Biomasse (1) auf die faserhaltige Biomasse (1) während einer Einwirkzeit;
Beaufschlagen (S32) der faserhaltigen Biomasse (1) mit Pansensaft (24); und
Zuführen (S34) der mit dem Pansensaft (24) beaufschlagten faserhaltigen Biomasse (1) zu der Biogasanlage (100).

2. Verfahren nach Anspruch 1, wobei das Sammeln (S12) der faserhaltigen Biomasse (1) ein Verpressen (S16) der faserhaltigen Biomasse (1) zu Ballen (10) umfasst, und wobei die mit dem Mittel (14) zum Aufschließen der faserhaltigen Biomasse (1) beaufschlagte faserhaltige Biomasse (1) zu Ballen (10) gepresst wird.

3. Verfahren nach Anspruch 2, wobei das Zuführen (S34) ein Zerkleinern (S30) der gepressten Ballen (10) an der Biogasanlage (100) umfasst.

4. Verfahren nach einem der vorhergehenden Ansprüche, wobei das Sammeln (S12) der faserhaltigen Biomasse (1) ein Häckseln (S14) umfasst, und wobei die faserhaltige Biomasse (1) vor und/oder während und/oder nach dem Beaufschlagen (S14) mit dem Mittel (14) zum Aufschließen der faserhaltigen Biomasse gehäckselt wird.

5. Verfahren nach einem der vorhergehenden Ansprüche, **dadurch gekennzeichnet, dass** das Bereitstellen (S10) und/oder das Sammeln (S12) der faserhaltigen Biomasse (1) auf einer landwirtschaftlichen Nutzfläche (200) erfolgt, auf der die faserhaltige Biomasse (1) entstanden ist, wobei das Bereitstellen (S10) umfasst:
Mähen der landwirtschaftlichen Nutzfläche (200), und/oder
Aufnehmen der lose auf der landwirtschaftlichen Nutzfläche (200) liegenden faserhaltigen Biomasse (1).

6. Verfahren nach einem der Ansprüche 2 bis 5, **dadurch gekennzeichnet, dass** das Häckseln (S14) der faserhaltigen Biomasse (1) durch einen Feldhäcksler bzw. das Verpressen (S16) der faserhaltigen Biomasse (1) zu Ballen (10) durch eine Ballenpresse (12) erfolgt.

7. Verfahren nach einem der vorstehenden Ansprüche, **dadurch gekennzeichnet, dass** das Sammeln (S12) mittels einer über die landwirtschaftliche Nutzfläche fahrenden oder gefahrenen Maschine (12) erfolgt, mit der eine Einrichtung (18) zum Beaufschlagen der faserhaltigen Biomasse (1) mit dem Mittel (14) zum Aufschließen der faserhaltigen Biomasse (1) mitgeführt wird.

8. Verfahren nach Anspruch 7, **dadurch gekennzeichnet, dass** das Beaufschlagen (S14) der faserhaltigen Biomasse (1) mit dem Mittel (14) zum Aufschließen der faserhaltigen Biomasse (1) aus einem an der Maschine (12) angeordneten und/oder mit der Maschine (12) mitgeführten Tank (16) erfolgt.

9. Verfahren nach einem der vorhergehenden Ansprüche, **dadurch gekennzeichnet, dass** die faserhaltige Biomasse (1) eine Cellulose-haltige Biomasse umfasst, vorzugsweise eine Lignocellulose-haltige Biomasse, besonders bevorzugt Stroh.

10. Verfahren nach einem der vorhergehenden Ansprüche, **dadurch gekennzeichnet, dass** das Mittel (14) zum Aufschließen der faserhaltigen Biomasse (1) eine Lauge ist, vorzugsweise Natronlauge.
